# EUROPEAN PATENT SPECIFICATION

(11) **EP 3 809 996 B1**
(45) Date of publication and mention of the grant of the patent: **17.04.2024**
(21) Application number: 19734486.4
(22) Date of filing: 21.06.2019
(51) Int. Cl.: A61B 17/54, A45D 29/00, A61B 17/32

(54) **SKIN TREATMENT APPARATUS**
HAUTBEHANDLUNGSVORRICHTUNG
APPAREIL DE TRAITEMENT DE LA PEAU

(30) Priority: 22.06.2018 GB 201810277
(43) Date of publication of application: 28.04.2021
(73) Proprietor: SCHOLL'S WELLNESS COMPANY LIMITED, Bracknell, Berkshire, RG12 1WA (GB)
(72) Inventor: LIU, Benjamin, Hull Humberside HU8 7DS (GB); OLIVER, Richard, Hull Humberside HU8 7DS (GB); SIMPSON, Adam, Hull Humberside HU8 7DS (GB); WITTY, Chris, Hull Humberside HU8 7DS (GB)
(74) Representative: Marks & Clerk LLP
(86) International application number: PCT/GB2019/051765
(87) International publication number: WO 2019/243844

(56) References cited:
- GB-A- 2 537 161
- US-A- 2 723 672
- US-A1- 2004 087 992
- US-A1- 2013 144 280
- US-B2- 7 226 457

## Description

The present invention relates to a skin treatment device; and, more particularly, to an electrical skin treatment device for delivering more than one benefit with a roller head. The roller head may be used to remove a hard, dry skin layer on a surface of the foot, for example, the heel, the sole or the toes. The roller head may also be used for other skin treatments such as skin exfoliation, moisturising and/or massaging.

Calluses and/or areas of hard skin occur on feet and can be caused by persistent rubbing or uneven pressure, for example from ill-fitting shoes. They are most commonly found at the heel, the ball of the foot and the sides of the toes. Calluses are often unsightly, and the thicker they are the more yellow they can look. With time, particularly thick calluses can become cracked and painful. There are a number of known methods for reducing or removing calluses and hard skin which include electrically operated foot file devices.

The known skin treatment devices provide a single benefit for each roller head. The user needs to change the roller head in order to receive more than one benefit from the skin treatment device.

US2723672A discloses a tool consisting of a rotary head having curved, tapered, sharply pointed cutting teeth that are integral with the head, the same being gouged out of the circular periphery of the head in circumferentially spaced relation, all curved in the direction of rotation and projecting from the otherwise smooth circular periphery of the head just far enough to do fine painless cutting that reduces the skin to a fine powder which drops off or is blown away with the air currents set up in the high speed rotation of the tool.

US2004/087992 discloses a system to deliver a composition, preferably a medical or pharmaceutical composition or active, through the stratum corneum of skin, without introducing bleeding or damage to tissue, and absent pain or other trauma. The dimensions and shapes of the microelements are controlled so as to control the penetration depth into the skin. The microelements can be "hollow" such that passageways are created therethrough to allow the composition to flow from a chamber, through the microelements, and into the skin. Alternatively, the microelements can be "solid," and the composition is applied directly to the skin just before or just after the microelements are applied to the skin surface to create the openings in the stratum corneum.

The present invention aims to provide multiple benefits with a single roller head, therefore mitigating the need for a user to switch roller heads in order to receive more than one benefit. According to the invention, there is provided a roller head for a skin treatment device according to claim 1. Other embodiments are listed in the dependent claims.

Aspects of the disclosure are also described in detail, by way of example only, with reference to the accompanying drawings, in which:
Embodiments will now be described with reference to the Figures.
Figure 1 shows a roller head having an abrasive surface with multiple peaks;
Figure 2 is a schematic illustration of the surface of a roller head having multiple peaks and an inset showing a single peak;
Figure 3 shows a set of roller heads having a surface with different numbers of peaks;
Figure 4 shows a roller head with multiple peaks with a space between the apex of the first and second peak; and
Figure 5 shows perspective and end on views of a roller head having multiple peaks formed from trapezoidal sections.

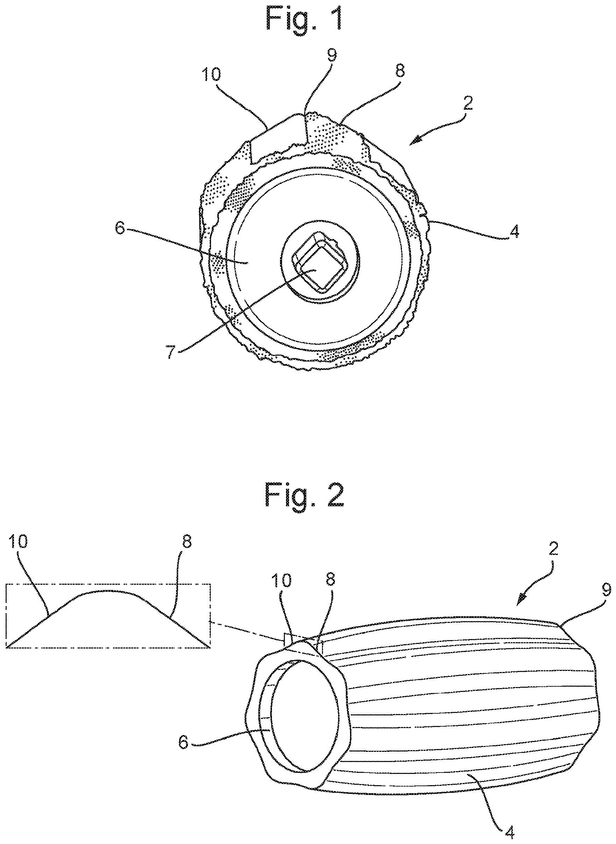

Figure 1 shows a roller head 2 having for a skin treatment device (not shown).

The roller head 2 comprises a drum 6 and abrasive surface 4. The drum 6 comprises a shaft 7 at each end of the drum 6. The drum 6 comprises at least one peak 9 which comprises a first surface 8 and a second surface 10.

The first surface 8 differs from the second surface 10 so that the first surface 8 provides a different benefit to the user compared to the second surface 10. For example, rubbing the first surface 8 on a user's skin may provide a different benefit by having a different effect on the user's skin compared to rubbing the second surface 10 on the user's skin. This allows a single roller head to be used for more than one benefit.

The roller head is configured to be rotated by a skin treatment device. The skin treatment device couples to the roller head via shaft 7. This allows the skin treatment device to rotate the drum along its longitudinal axis. The rotation of the drum along its longitudinal axis allows a user to provide the benefit to the user's skin.

The user applies the first benefit from the first surface 8 and the second benefit from the second surface 10 by rotating the roller head in different directions along the longitudinal axis so that the first surface 8 comes into contact with the user's skin in one direction and the second surface 10 comes into contact with the user's skin in another direction. For example, when the roller head 2 is rotated in a clockwise direction the first surface 8 comes into contact with the user's skin and when the roller head 2 is rotated in an anticlockwise direction the second surface 10 comes into contact with the user's skin.

The benefit provided to the user's skin by the roller head may include removal of hard skin, skin smoothing, exfoliation, massaging, moisturisation and/or lubrication.

Figure 2 shows an illustration of the surface of the roller head 2 having a drum 6 and an abrasive surface 4 for removal of hard skin. The roller head of Figure 2 may be used to provide the benefit of removing hard skin.

Hard skin is removed using a roller head having a rough surface and the movement of the rough surface of the roller head on the skin removes the hard skin. The degree of roughness of surface of the roller head alters the rate at which hard skin is removed. For example, a roller head with a rougher surface will remove hard skin at a more quickly than a roller head with a less rough surface. A user may want to initially use a roller head having rougher surface and then use a less rough roller head to achieve optimum hard skin removal. The examples set out below may provide a convenient method for a user to achieve the required level of hard skin removal.

In the example illustrated in Figure 2 the abrasive surface 4 of the roller head 2 comprises seven peaks 9 with those peaks providing seven first surfaces 8 having a first roughness and seven second surfaces 10 having a second roughness. The inset of Figure 2 shows a single peak 9 with a first surface and second surface is shown in more detail.

In use, the user applies the first benefit from the first surface 8 to their skin by rotating the roller head 2 in a clockwise direction. As the roller head 2 is rotated clockwise, the first surface 8 of each peak sequentially comes into contact with the user's skin. As the first surfaces 8 of the roller contacts the user's skin the first benefit is applied to the user's skin. When the roller head is rotated in an anti-clockwise direction the second surface 10 of each peak 9 sequentially comes into contact with the user's skin and the second benefit is applied to the user's skin.

The example in Figure 2 shows a roller with an abrasive surface 4. In this example, the first surface 8 has a different roughness to the second surface 10.

The roller head 2 may be used in conjunction with a skin treatment device having an electro mechanical mechanism. The roller head 2 may be inserted into the skin treatment device and may be coupled so that the electro mechanical mechanism of the skin treatment device rotates the roller head. In an example, the skin treatment device comprises two arms and the shaft 7 of the roller head is held by the two arms of the skin treatment device. The skin treatment device is configured to rotate the roller head by transferring the rotation of the electro mechanical mechanism to the roller head via the shaft 7.

The user's experience whilst using the roller head is dependent upon the number of peaks. A smaller number of peaks provides a larger surface area for each first surface 8 and second surface 10. The contact between the user's skin and each first surface 8 and each second surface 10 will be increased with a smaller number of peaks. This leads to a greater difference between the first benefit and the second benefit. A larger number of peaks provides a more circular roller head and a more circular roller head may increase the comfort for the user during use. The number of peaks are selected to provide a user with a larger difference between the first benefit and the second benefit and provide a roller head that is comfortable during use. In a preferred example, the roller head has between 4 and 8 peaks, for example between 5 and 7 peaks. The roller head may also have 5 peaks, 6 peaks and/or 7 peaks.

In the examples illustrated in Figures 1 and 2 the roller head comprises a peak having a continuous surface. For example, the roller head has a convex peak followed by a concave trough. The gradient of the surface of the roller between the convex peak and concave trough is continuous.

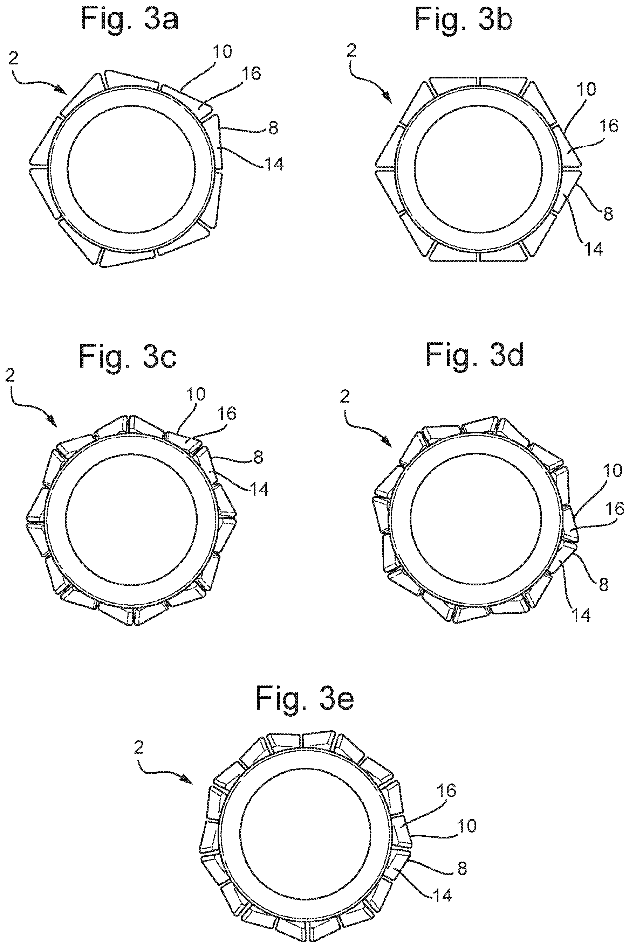

The surface of the roller heads illustrated in Figure 3 comprise a discontinuous surface. The roller head comprises a series of first quadrilateral portions 14 and second quadrilateral portions 16 arranged to form peaks. The first quadrilateral portion 14 forms a first surface 8 and the second quadrilateral portion 16 forms a second surface 10 and, as discussed above, the first surface 8 provides a first benefit to the user's skin and the second surface 10 provides a second benefit to the user's skin.

In the example illustrated in Figure 3, the first quadrilateral portion 14 and second quadrilateral portion 16 vary in height to form an angled surface which corresponds to the first surface 8 and second surface 10. The first quadrilateral portion 14 is inverted with respect to the second quadrilateral portion 16 to form the peak and this arrangement of first and second quadrilateral portions leads to the first surface 8 having a different angle with respect to the roller than the second surface 10.

Figure 3 shows cross sectional views of roller heads having different numbers of peaks. Figure 3 (a) shows a roller head having 5 peaks with those peaks providing 5 first surfaces delivering a first benefit to the user's skin and 5 second surfaces delivering a second benefit. Figure 3(b) shows a roller head having 6 peaks with 6 first surfaces delivering a first benefit to the user's skin and 6 second surfaces delivering a second benefit. Figure 3 (c) shows a roller head having 8 peaks with 8 first surfaces delivering a first benefit to the user's skin and 8 second surfaces delivering a second benefit. Figure 3 (d) shows a roller head having 9 peaks with 9 first surfaces delivering a first benefit to the user's skin and 9 second surfaces delivering a second benefit.

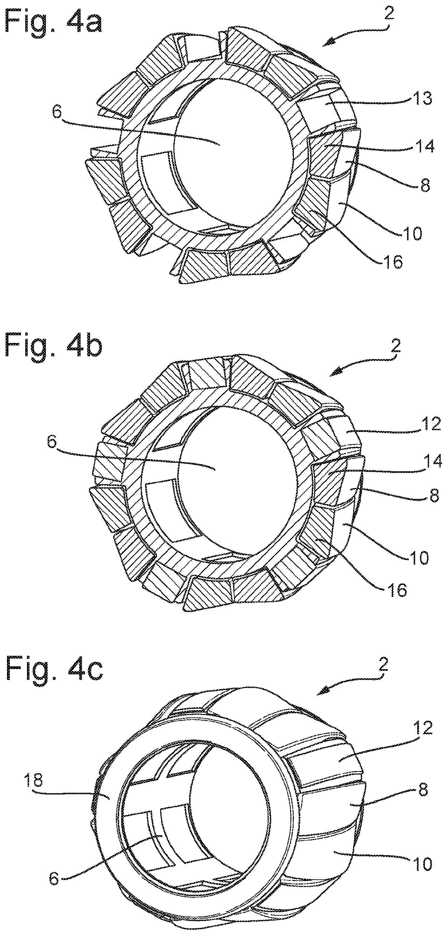

Figure 4 shows the outer surface of a roller head having a first surface 8 to provide a first benefit, a second surface 10 to provide a second benefit and additional portions 12 that to provide a further benefit to the user. The roller head has a discontinuous surface and similar to the roller head described in Figure 3 has a first quadrilateral portion 14 and a second quadrilateral portion 16 and the first quadrilateral portion 14 is inverted with respect to the second quadrilateral portion 16 to form the first surface 8 and second surface 10 respectively.

Figure 4(a) shows part of a roller head with slots 13 between the first quadrilateral portion 14 and the second quadrilateral portion 16. The slots 13 may be used to insert an additional portion 12. Figure 4(b) shows the roller head of Figure 4(a) with the additional portions 12 located in the slots 13. The additional portions 12 may provide a further benefit in addition to the first benefit provided by the first surface 8 and the second benefit provided by the second surface 10. Figure 4(c) shows the roller head with a cap 18 located at each end of the roller head 2. The caps 18 at each end may fix the first quadrilateral portion 14, second quadrilateral portion 16 and additional portions 12 in place on the roller head.

In the example illustrated in Figure 4 the first quadrilateral portion 14, second quadrilateral portion 16 and additional portions 12 are held in place on the roller head via caps 18. In other examples, the first quadrilateral portion 14, second quadrilateral portion 16 and additional portions 12 may be held in place without caps 18 at each end of the roller. For example, the roller head 2 may be formed by moulding with the first quadrilateral portion 14, second quadrilateral portion 16 and additional portions 12 held in place during moulding.

In the example illustrated in Figure 4, the addition portions are flat and located between the highest point on the roller head surface of the first and second quadrilateral portion. This configuration allows each additional portion to come into contact with the skin as the roller head is rotated in either a clockwise or anticlockwise direction.

The additional portions may be used to provide the user with the benefit of moisturisation, hard skin removal, lubrication, massaging, and/or exfoliation. For example, the first quadrilateral portion 14 may be used to remove hard skin, the second quadrilateral portion 16 may be used to smooth the hard skin and additional portions 12 may be used to lubricate the skin.

In the examples illustrated in Figure 4 each quadrilateral portion comprise rounded edges.

The additional portion 12 may also be fixed to the roller head. The additional portion may also be an integral part of the drum.

In the examples illustrated in Figure 1 and 2 the roller head comprises a first surface 8 to provide a first benefit and a second surface 10 to provide a second benefit. The roller head may also comprise one or more additional surfaces to provide one or more additional benefits. An additional surface may be located at the apex of the peak between the first surface 8 and the second surface 10. For example, the additional surface may have a different angle on the surface of the roller head to the first surface 8 and the second surface 10. The additional surface may be flat. The additional surface may provide the user with the benefit of moisturisation, hard skin removal, lubrication, massaging, and/or exfoliation

In the examples illustrated above, the first surfaces 8 provide a first benefit and the second surfaces 10 provide a second benefit. The first surfaces 8 may provide more than one benefit and/or the second surfaces may provide more than one benefit. For example, the first surfaces and second surfaces may vary along the circumference of the roller head.

The additional portion, first surface and second surface may be used to different benefits to the user's skin. Two or more of the additional portion, first surface and second surface may also be used to provide the same benefit to the user's skin.

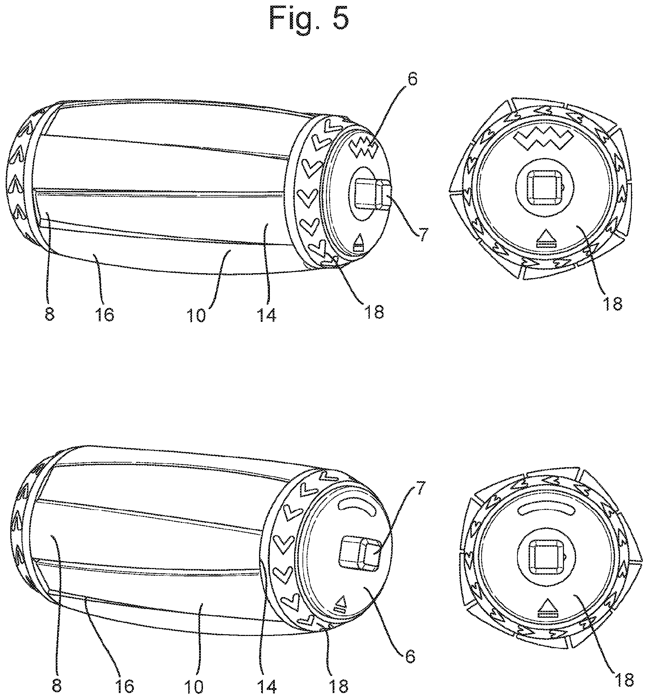

Figure 5 shows a roller head having a first portion 14 and a second portion 16. Similar to the roller heads described previously, the roller head in Figure 5 comprises a drum 6 and a shaft 7. The first portion 14 and second portion 16 form peaks with a first surface 8 and a second surface 10. As illustrated, the first surface 8 and second surface 10 have trapezium shaped outer facing sections i.e. the surface has is quadrilateral with two parallel sides. In the example illustrated, two trapezium sections of the first surface 8 and the second surface 10 form a single peak. For example, one trapezium section forms first surface 8 and the other trapezium section forms second surface 12.

The roller head may have a series of first surfaces 8 and the second surfaces 10 having trapezium sections. For example, each first surface 8 and the second surface 10 formed by trapezium sections form a single peak and the roller head has multiple peak using a series of trapezium sections. The adjacent trapezium sections may slot into one another.

As illustrated in Figure 5, the roller head may have indicators on caps 18 that show the user the orientation of the roller head required to achieve a first benefit and a second benefit. In an example, the roller head may have arrows, colour indications, writing and/or symbols on the surface that indicate the benefit provided by rotating the roller head in a given direction.

The trapezoidal sections illustrated in Figure 5 are isosceles trapezoids having two sides with equal length and one base larger than the other base. In other examples the trapezium sections may take other shapes, for example the sections may have a rectangular shape and/or a triangular shape. In an example, a roller head may have sections with more than one shape, for example a combination of a rectangular section and a trapezium section, a rectangular section and a triangular section, and a trapezium section and a triangular section.

In the example illustrated in Figure 5 the first surface 8 and second surface 10 has a rounding on one of edges and the other edge is flat so that it is flush with the adjacent surface (e.g. the first surface 8 is rounded on an edge adjacent the one second surface 10 and flat on an edge adjacent to the other second surface 10). The rounding is located so that rounded edge contacts the skin before the straight edge as the roller head rotates in use. The rounding on the edge that first makes contact with the skin as the roller head rotates increases the area of the section coming into contact with the skin as the roller head rotates and therefore increase efficacy of the roller head.

In an example, the first portion 14 is coupled to one cap 18 and the second portion 16 is coupled to another cap 18. The roller head having peaks is formed by slotting the first portion 14 into the second portion 16 by moving one cap 18 towards the other cap 18. In the examples illustrated the roller head has a convex shape. In other examples the roller head may have a concave shape. The roller head may also have one end with a smaller diameter than the other end.

Further modifications and developments can be made without departing from the scope of the invention described herein.

## Claims

1. A roller head (2) for a skin treatment device configured to drive the roller head by an electro-mechanical mechanism;
a surface (4) of the roller head comprises at least one peak (9), the at least one peak is arranged so that, when in use, a first surface (8) of the peak (9) contacts with the skin with when the roller head (2) rotated in a clockwise direction and a second surface (10) of the peak (9) contacts with the skin with when the roller head (2) rotated in an anticlockwise direction
wherein the first surface (8) differs from the second surface (10) and the first surface (8) is configured to provide a first benefit and the second surface (10) is configured to provide a second benefit
wherein the roller head (2) further comprising an additional surface configured to provide a further benefit when the roller head (2) is rotated clockwise and anticlockwise.

2. The roller head of claim 1, wherein the first surface (8) and/or second surface (10) is configured for removal of hard skin, skin smoothing, exfoliation, massaging, moisturisation and/or lubrication.

3. The roller head of claim 2, wherein the roller head (2) is configured to remove hard skin and the first surface (8) has a first roughness and the second surface (10) has a second roughness.

4. The roller head of claim 1, wherein the additional surface is configured to provide the further benefit of removal of hard skin, skin smoothing, exfoliation, massaging, moisturisation and/or lubrication.

5. The roller head of claim 4, wherein the additional surface differs from the first surface and/or second surface.

6. The roller head of claim 5, wherein the additional surface is configured to provide lubrication and/or moisturisation.

7. The roller head of claims 1 to 4, wherein the roller head (2) comprises a first quadrilateral portion (14) that forms the first surface (8) and a second quadrilateral portion (16) that forms the second surface (10);
wherein the first quadrilateral portion (14) and second quadrilateral portions (16) are arranged to form the at least one peak (9).

8. The roller head of claim 6 or 7, further comprising an additional portion (12) that provides a further benefit.

9. The roller head of claim 8, wherein the additional portion (12) is configured to provide the further benefit of removal of hard skin, skin smoothing, exfoliation, massaging, moisturisation and/or lubrication

10. The roller head of any preceding claim, wherein the roller head (2) comprises 4 to 10 peaks (9), for example 4 to 8 peaks, for example 5 to 7 peaks.

11. A skin treatment device comprising a roller head (2) according to Claim 1.

12. The skin treatment device of claim 11, wherein the roller head (2) is according to any one of claims 2 to 10.

## Patentansprüche

1. Walzenkopf (2) für eine Hautbehandlungsvorrichtung, die dafür konfiguriert ist, den Walzenkopf durch einen elektromechanischen Mechanismus anzutreiben,
wobei eine Oberfläche (4) des Walzenkopfs mindestens eine Spitze (9) umfasst, wobei die mindestens eine Spitze des Walzenkopfs so angeordnet ist, dass, wenn sie in Benutzung ist, eine erste Fläche (8) der Spitze (9) in Kontakt mit der Haut tritt, wenn der Walzenkopf (2) im Uhrzeigersinn gedreht wird, und eine zweite Fläche (10) der Spitze (9) in Kontakt mit der Haut tritt, wenn der Walzenkopf (2) gegen den Uhrzeigersinn gedreht wird,
wobei sich die erste Fläche (8) von der zweiten Fläche (10) unterscheidet und die erste Fläche (8) dafür konfiguriert ist, einen ersten Nutzen bereitzustellen und die zweite Fläche (10) dafür konfiguriert ist, einen zweiten Nutzen bereitzustellen,
wobei der Walzenkopf (2) ferner eine zusätzliche Fläche umfasst, die dafür konfiguriert ist, einen weiteren Nutzen bereitzustellen, wenn der Walzenkopf (2) im Uhrzeigersinn und gegen den Uhrzeigersinn gedreht wird.

2. Walzenkopf nach Anspruch 1, wobei die erste Fläche (8) und/oder die zweite Fläche (10) zum Entfernen von harter Haut, Hautglätten, Exfolieren, Massieren, Befeuchten und/oder Einfetten konfiguriert ist.

3. Walzenkopf nach Anspruch 2, wobei der Walzenkopf (2) dafür konfiguriert ist, harte Haut zu entfernen, und die erste Fläche (8) eine erste Rauheit aufweist und die zweite Fläche (10) eine zweite Rauheit aufweist.

4. Walzenkopf nach Anspruch 1, wobei die zusätzliche Fläche dafür konfiguriert ist, den weiteren Nutzen des Entfernens von harter Haut, Hautglättens, Exfolierens, Massierens, Befeuchtens und/oder Einfettens bereitzustellen.

5. Walzenkopf nach Anspruch 4, wobei sich die zusätzliche Fläche von der ersten Fläche und/oder der zweiten Fläche unterscheidet.

6. Walzenkopf nach Anspruch 5, wobei die zusätzliche Fläche dafür konfiguriert ist, Befeuchten und/oder Einfetten bereitzustellen.

7. Walzenkopf nach einem der Ansprüche 1 bis 4, wobei der Walzenkopf (2) einen ersten vierseitigen Abschnitt (14), der die erste Fläche (8) bildet, und einen zweiten vierseitigen Abschnitt (16), der die zweite Fläche (10) bildet, umfasst,
wobei der erste vierseitige Abschnitt (14) und der zweite vierseitige Abschnitt (16) angeordnet sind, um die mindestens eine Spitze (9) zu bilden.

8. Walzenkopf nach Anspruch 6 oder 7, der ferner einen zusätzlichen Abschnitt (12) umfasst, der einen weiteren Nutzen bereitstellt.

9. Walzenkopf nach Anspruch 8, wobei der zusätzliche Abschnitt (12) dafür konfiguriert ist, den weiteren Nutzen des Entfernens von harter Haut, Hautglättens, Exfolierens, Massierens, Befeuchtens und/oder Einfettens bereitzustellen.

10. Walzenkopf nach einem der vorhergehenden Ansprüche, wobei der Walzenkopf (2) 4 bis 10 Spitzen (9), zum Beispiel 4 bis 8 Spitzen, zum Beispiel 5 bis 7 Spitzen, umfasst.

11. Hautbehandlungsvorrichtung, die einen Walzenkopf (2) nach Anspruch 1 umfasst.

12. Hautbehandlungsvorrichtung nach Anspruch 11, wobei der Walzenkopf (2) nach einem der Ansprüche 2 bis 10 ist.

## Revendications

1. Tête de rouleau (2) pour un dispositif de traitement de la peau configuré pour entraîner la tête de rouleau par un mécanisme électromécanique ;
une surface (4) de la tête de rouleau comprend au moins une pointe (9), l'au moins une pointe étant agencée de sorte que, en cours d'utilisation, une première surface (8) de la pointe (9) entre en contact avec la peau lorsque la tête de rouleau (2) tourne dans le sens des aiguilles d'une montre, et une deuxième surface (10) de la pointe (9) entre en contact avec la peau lorsque la tête de rouleau (2) tourne dans le sens contraire des aiguilles d'une montre ;
dans laquelle la première surface (8) diffère de la deuxième surface (10), et la première surface (8) est configurée pour fournir un premier avantage et la deuxième surface (10) est configurée pour fournir un deuxième avantage ;
dans laquelle la tête de rouleau (2) comprend en outre une surface supplémentaire configurée pour fournir un avantage supplémentaire lorsque la tête de rouleau (2) tourne dans le sens des aiguilles d'une montre et dans le sens contraire des aiguilles d'une montre.

2. Tête de rouleau selon la revendication 1, dans laquelle la première surface (8) et/ou la deuxième surface (10) sont configurées pour l'élimination de callosités, le lissage de la peau, l'exfoliation, le massage, l'hydratation et/ou la lubrification.

3. Tête de rouleau selon la revendication 2, dans laquelle la tête de rouleau (2) est configurée pour éliminer les callosités, et la première surface (8) a une première rugosité et la deuxième surface (10) a une deuxième rugosité.

4. Tête de rouleau selon la revendication 1, dans laquelle la surface supplémentaire est configurée pour offrir l'avantage supplémentaire de l'élimination des callosités, du lissage de la peau, de l'exfoliation, du massage, de l'hydratation et/ou de la lubrification.

5. Tête de rouleau selon la revendication 4, dans laquelle la surface supplémentaire diffère de la première surface et/ou de la deuxième surface.

6. Tête de rouleau selon la revendication 5, dans laquelle la surface supplémentaire est configurée pour assurer une lubrification et/ou une hydratation.

7. Tête de rouleau selon les revendications 1 à 4, dans laquelle la tête de rouleau (2) comprend une première partie quadrilatère (14) qui forme la première surface (8) et une deuxième partie quadrilatère (16) qui forme la deuxième surface (10) ;
dans laquelle la première partie quadrilatère (14) et la deuxième partie quadrilatère (16) sont agencées pour former ladite au moins une pointe (9).

8. Tête de rouleau selon les revendications 6 ou 7, comprenant en outre une partie supplémentaire (12) qui fournit un avantage supplémentaire.

9. Tête de rouleau selon la revendication 8, dans laquelle la partie supplémentaire (12) est configurée pour offrir l'avantage supplémentaire d'éliminer les callosités, de lisser la peau, d'exfolier, de masser, d'hydrater et/ou de lubrifier.

10. Tête de rouleau selon l'une quelconque des revendications précédentes, dans laquelle la tête de rouleau (2) comprend 4 à 10 pointes (9), par exemple 4 à 8 pointes, par exemple 5 à 7 pointes.

11. Dispositif de traitement de la peau comprenant une tête de rouleau (2) selon la revendication 1.

12. Dispositif de traitement de la peau selon la revendication 11, dans lequel la tête de rouleau (2) est conforme à l'une quelconque des revendications 2 à 10.
